(19) Europäisches Patentamt
European Patent Office
Office européen des brevets

(11) Veröffentlichungsnummer : **0 046 917**
**B1**

(12) # EUROPÄISCHE PATENTSCHRIFT

(45) Veröffentlichungstag der Patentschrift : **07.12.83**

(51) Int. Cl.³ : **C 07 C119/048**, C 07 C118/02, C 08 G 18/76

(21) Anmeldenummer : **81106367.6**

(22) Anmeldetag : **17.08.81**

(54) **Neue Polyisocyanatsgemische der Diphenylmethanreihe, sowie ihre Verwendung als Aufbaukomponente bei der Herstellung von Polyurethankunststoffen nach dem Isocyanat-Polyadditionsverfahren.**

(30) Priorität : **28.08.80 DE 3032358**

(43) Veröffentlichungstag der Anmeldung : **10.03.82 Patentblatt 82/10**

(45) Bekanntmachung des Hinweises auf die Patenterteilung : **07.12.83 Patentblatt 83/49**

(84) Benannte Vertragsstaaten : **AT BE CH DE FR GB IT LI NL SE**

(56) Entgegenhaltungen :
**EP-A- 0 024 665**
**DE-A- 2 452 714**
**DE-B- 1 959 462**
**US-A- 3 933 701**
**Die Akte enthält technische Angaben, die nach dem Eingang der Anmeldung eingereicht wurden und die nicht in dieser Patentschrift enthalten sind.**

(73) Patentinhaber : **BAYER AG**
**Zentralbereich Patente, Marken und Lizenzen**
**D-5090 Leverkusen 1, Bayerwerk (DE)**

(72) Erfinder : **Knöfel, Hartmut, Dr.**
**Dülmener Weg 21**
**D-5068 Odenthal-Erberich (DE)**
Erfinder : **Brockelt, Michael**
**Neuenhauser Weg 1**
**D-5060 Bergisch-Gladbach 2 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 046 917 B1

# Neue Polyisocyanatgemische der Diphenylmethanreihe, sowie ihre Verwendung als Aufbaukomponente bei der Herstellung von Polyurethankunststoffen nach dem Isocyanat-Polyadditionsverfahren

Die Erfindung betrifft neue Gemische von Polyisocyanaten der Diphenylmethanreihe, deren Hauptkomponenten insbesondere durch Isocyanatgruppen in 3,4'- und gegebenenfalls in 3,2'-Stellung gekennzeichnet sind, sowie ihre Verwendung als Isocyanatkomponente bei der Herstellung von Polyurethankunststoffen nach dem Isocyanat-Polyadditionsverfahren.

Zu den technisch und wirtschaftlich wichtigsten Polyisocyanaten der Polyurethanchemie gehören neben den difunktionellen Diisocyanato-toluol-Isomeren (TDI) und den difunktionellen Diisocyanatodiphenylmethan-Isomeren (insbesondere 2,4'- und/oder 4,4'-Diisocyanatodiphenylmethan) vor allem auch höherfunktionelle Polyisocyanatgemische der Diphenylmethanreihe, die neben den zuletzt genannten Diisocyanatodiphenylmethan-Isomeren noch höherkernige, höher als difunktionelle Polyisocyanate der Diphenylmethanreihe enthalten, und die in an sich bekannter Weise durch Phosgenierung von Anilin/Formaldehyd-Kondensaten zugänglich sind. Diese zuletzt genannten Polyisocyanatgemische der Diphenylmethanreihe, die im statistischen Mittel eine über 2 liegende NCO-Funktionalität aufweisen, stellen insbesondere wichtige Ausgangsmaterialien zur Herstellung von Polyurethanschaumstoffen dar. Die Gemische des Standes der Technik sind jedoch mit einigen gravierenden Nachteilen behaftet. Es handelt sich nämlich stets um Gemische von destillierbaren, niederfunktionellen Polyisocyanaten mit nicht-destillierbaren höherfunktionellen Homologen, bei denen eine Erhöhung der NCO-Funktionalität stets mit einer Molekülvergrößerung und damit einer Viskositätserhöhung des Gemisches erkauft werden muß. Ein weiterer Nachteil der höherfunktionellen Polyisocyanatgemische der Diphenylmethanreihe gemäß Stand der Technik ist in deren Tendenz zu sehen, bei niedrigen Temperaturen teilweise zu kristallisieren, so daß oftmals eine thermische oder chemische Behandlung der Gemische zwecks ihrer Verflüssigung erforderlich ist. Schließlich können nach den Methoden der klassischen « MDI-Chemie » nur schwer chemisch weitgehend reine Polyisocyanate einer um 3 liegenden NCO-Funktionalität hergestellt werden, es sei denn, man nimmt bei der im Prinzip möglichen destillativen Abtrennung von trifunktionellen Polyisocyanaten aus den Phosgenierungsprodukten in Kauf, daß gleichzeitig ganz erhebliche Mengen an Destillationsrückständen anfallen, die neben höher als trifunktionellen Polyisocyanaten insbesondere Carbodiimid- und Isocyanuratgruppen aufweisende Folgeprodukte enthalten, und die wegen ihrer hohen Viskosität und wegen ihres Gehaltes an oftmals unerwünschten Nebenprodukten der genannten Art zur Herstellung von hochwertigen Polyurethankunststoffen nur bedingt geeignet sind.

Durch die Bereitstellung der nachstehend näher beschriebenen Polyisocyanatgemische werden diese Nachteile der Polyisocyanatgemische der Diphenylmethanreihe gemäß Stand der Technik weitgehend beseitigt.

Di- und Triisocyanato-diphenylmethane, die gegebenenfalls methyl- oder Ethylsubstituenten aufweisen, werden bereits in der US-A-3 933 701 beschrieben. Es muß jedoch hervorgehoben werden, daß

a) alle in dieser Vorveröffentlichung genannten, Alkylsubstituenten aufweisenden Polyisocyanate die NCO-Gruppen in den « Klassischen » 2-, 2'-, 4- und 4'-Stellungen und nicht etwa in der bei technischen Polyisocyanaten der Diphenylmethanreihe unüblichen 3-Stellung aufweisen,

b) von Gemischen der nachstehend näher beschriebenen erfindungsgemäßen Art mit einer durchschnittlichen NCO-Funktionalität von 2,2 bis 2,7 in der Vorveröffentlichung nicht die Rede ist und

c) die Verbindungen der Entgegenhaltung durch eine Friedel-Crafts-Reaktion unter Verwendung von Isocyanatgruppen aufweisenden Benzylchloriden hergestellt werden, was selbstverständlich wegen der Empfindlichkeit der Isocyanatgruppen zu ganz beträchtlichen Ausbeuteverlusten führen muß, so daß die in der Vorveröffentlichung empfohlene Herstellung der Polyisocyanate kaum ein technisch brauchbarer Weg darstellen dürfte.

Die DE-B-1 959 462 betrifft spezielle Tetraisocyanate, die zur Modifizierung von Klebstoffen Verwendung finden können. Polyisocyanatgemische der nachstehend näher beschriebenen erfindungsgemäßen Art werden in dieser Vorveröffentlichung auch nicht andeutungsweise angesprochen.

Gegenstand der Erfindung sind, gegebenenfalls im Gemisch mit bis zu 40 Gew.-%, bezogen auf Gesamtgemisch, an anderen alkylsubstituierten Polyisocyanatodiphenylmethan-Isomeren vorliegende Gemische von Polyisocyanaten der Formel

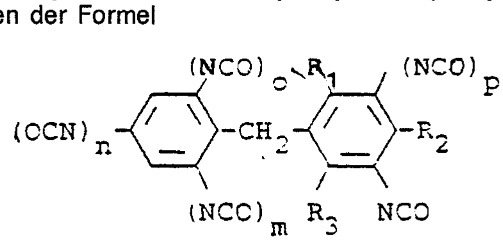

in welcher jeder der beiden aromatischen Ringe mindestens eine Isocyanatgruppe aufweist und wobei

$R_1$, $R_2$ und $R_3$ jeweils gleich oder verschieden sind und Wasserstoff oder eine Methyl- oder Ethylgruppe bedeuten, mit der Einschränkung, daß jeweils zwei der Reste $R_1$, $R_2$ und $R_3$ für Wasserstoff stehen und

m, n, o und p jeweils für 0 oder 1 stehen, wobei im Falle von m, n, o und/oder p = 0 die freie Valenz durch ein Wasserstoffatom abgesättigt ist, und wobei die Summe m + n + o + p im statistischen Mittel 1,2 bis 1,7 beträgt.

Gegenstand der Erfindung ist auch die Verwendung der neuen Polyisocyanatgemische als Isocyanatkomponente bei der Herstellung von Polyurethankunststoffen nach den Isocyanat-Polyadditionsverfahren.

Die oben und nachstehend gemachten Ausführungen bezüglich der Zusammensetzungen der erfindungsgemäßen Gemische, sowie der Ausgangsmaterialien und Zwischenprodukte beziehen sich auf gaschromatographisch ermittelbare Werte. Bei den erfindungsgemäßen Gemischen von Polyisocyanaten handelt es sich um Polyisocyanate der Diphenylmethanreihe, die einen Alkylsubstituenten mit 1 bis 2 Kohlenstoffatomen aufweisen, d. h. deren Alkylsubstituent einen Methyl- oder Ethylrest darstellt. Demzufolge steht im folgenden bei der Beschreibung der erfindungsgemäßen Gemische und auch bei der Beschreibung der bei den Verfahren zur Herstellung der erfindungsgemäßen Gemische eingesetzten Ausgangsmaterialien der Begriff « Alkyl- » stets für einen Substituenten der genannten Art. Dies gilt insbesondere für die gegebenenfalls Nitro-substituierten Alkylbenzole, die bei den einzelnen Verfahren als Ausgangsmaterialien eingesetzt werden können.

Das erste Verfahren zur Herstellung von erfindungsgemäßen Polyisocyanaten bzw. Polyisocyanatgemischen ist dadurch gekennzeichnet, daß man

a) ein 4-Nitrobenzylhalogenid in Gegenwart von Friedel-Crafts-Katalysatoren mit 1-Alkyl-2-nitrobenzol und/oder 1-Alkyl-4-nitro-benzol und/oder technischen Gemischen dieser Alkyl-nitro-benzol-Isomeren mit bis zu 15 Gew.-%, bezogen auf Gesamtgemisch, an 1-Alkyl-3-nitro-benzol, wobei die Alkylreste jeweils 1 bis 2 Kohlenstoffatome aufweisen, zu Dinitro-Verbindungen bzw. Gemischen von Dinitro-Verbindungen der Formel

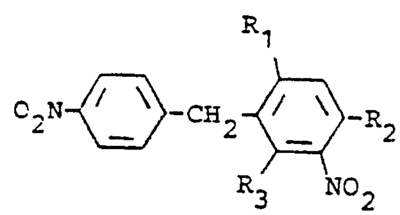

gegebenenfalls im Gemisch mit bis zu 20 Gew.-% an isomeren Dinitro-diphenylenmethanen umsetzt, das Umsetzungsprodukt von eingesetztem Katalysator befreit,

b) das gemäß a) erhaltene Reaktionsprodukt einer Nitrierungsreaktion unter Bildung von im wesentlichen Nitroverbindungen der Formel

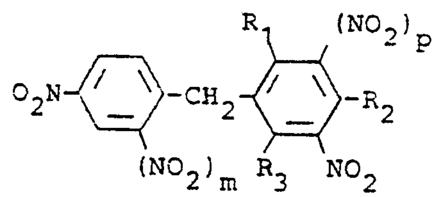

unterzieht, wobei in den unter a) und b) genannten Formeln $R_1$, $R_2$ und $R_3$ die obengenannte Bedeutung haben, und wobei m und p jeweils für 0 oder 1 stehen, wobei im Falle von m oder p = 0 die freie Valenz durch ein Wasserstoffatom abgesättigt ist, und wobei die Summe m + p im statistischen Mittel größer als 0 ist,

c) das gemäß b) erhaltene Umsetzungsprodukt durch Hydrierung oder Reduktion der Nitrogruppen in die entsprechenden aromatischen Diaminoverbindungen überführt und

d) die gemäß c) erhaltenen Polyaminoverbindungen durch Phosgenierung in die Polyisocyanate überführt,

wobei man gegebenenfalls

e) den Gehalt der gemäß c) erhaltenen Polyamingemische an Triamino-Isomeren vor der Phosgenierungsreaktion und/oder den Gehalt der gemäß d) erhaltenen Polyisocyanatgemische an dem entsprechenden Triisocyanato-Isomeren durch destillative Abtrennung von niedriger bzw. höher siedenden Nebenprodukten erhöht.

Unter « Nitrobenzylhalogenid » bzw. « Benzylhalogenid » sind hier und nachstehend vor allem die entsprechenden Benzylchloride bzw. -bromide, insbesondere die entsprechenden Benzylchloride zu verstehen.

Bei der Stufe a) des ersten Verfahrens erfolgt eine Friedel-Crafts-Kondensation zwischen 4-Nitrobenzylhalogenid und 1-Alkyl-2-nitro-benzol und/oder 1-Alkyl-4-nitro-benzol und/oder technischen Gemischen dieser Isomeren mit bis zu 15 Gew.-%, bezogen auf Gesamtgemisch, an 1-Alkyl-3-nitro-benzol,

wobei die Reaktionspartner in solchen Mengen zum Einsatz gelangen, daß für jedes Mol Nitrobenzylhalogenid 1,0 bis 20, vorzugsweise 2 bis 10 Mol, nitro-benzol und/oder Alkyl-nitro-benzol zur Verfügung stehen. Die im Überschuß eingesetzte Komponente dient dabei gleichzeitig als Lösungsmittel. Als Katalysatoren dienen die üblichen Friedel-Crafts-Katalysatoren, d. h. z. B. Aluminiumchlorid, Eisentrichlorid, Titantetrachlorid oder Zinntetrachlorid. Vorzugsweise wird Eisentrichlorid als Katalysator verwendet. Die Katalysatoren kommen im allgemeinen in Mengen von 1 bis 100, vorzugsweise 5 bis 50 Mol-%, bezogen auf die Benzylhalogenidkomponente zum Einsatz. Die Umsetzung wird im allgemeinen bei einer zwischen Raumtemperatur und dem Siedepunkt des Reaktionsgemisches, d. h. bei ca. + 20 bis ca. 200 °C, vorzugsweise bei 30 bis 120 °C liegenden Temperatur durchgeführt. Im Anschluß an die Kondensationsreaktion wird der Katalysator vorzugsweise durch Auswaschen z. B. mit Wasser und gegebenenfalls verdünnter Salzsäure entfernt und das überschüssige, nicht-umgesetzte Ausgangsmaterial abdestilliert.

Das gemäß a) erhaltene, vom Katalysator befreite Kondensationsprodukt wird anschließend in einer Stufe b) in an sich bekannter Weise einer Nitrierung unterworfen. Diese Nitrierung kann z. B. in Gegenwart eines geeigneten Lösungsmittelt wie beispielsweise Methylenchlorid erfolgen. Zur Nitrierung wird « Nitriersäure », d. h. ein Gemisch aus konzentrierter Schwefelsäure mit Salpetersäure oder, vorzugsweise, hochkonzentrierte, ca. 98 %ige Salpetersäure eingesetzt.

Die Menge der Nitriersäure wird so bemessen, daß für jedes Mol an gemäß a) erhaltener Dinitroverbindung mindestens 0,1 Mol, vorzugsweise 0,1 bis 2,1 Mol und insbesondere 0,2 bis 1,2 Mol Salpetersäure zur Verfügung stehen, so daß bei der Nitrierung in die Dinitroverbindung im statistischen Mittel mindestens 0,1, vorzugsweise 0,1 bis 1 weitere Nitrogruppen eingeführt werden, so daß eine $NO_2$-Funktionalität von 2,2 bis 2,7 resultiert. Die Nitrierungsreaktion wird im allgemeinen im Temperaturbereich zwischen − 20 und + 100 °C, insbesondere innerhalb des Bereichs von + 20 bis + 70 °C durchgeführt. Anschließend wird die nach der Nitrierungsreaktion vorliegende organische Phase von der Säure durch Phasentrennung, Auswaschen mit Wasser und beispielsweise einer Base wie Natriumcarbonat-Lösung beschreibt. Schließlich erfolgt die destillative Entfernung des gegebenenfalls verwendeten Hilfslösungsmittels, gegebenenfalls unter anschließendem Austreiben von Lösungsmittelresten mittels Wasserdampfdestillation.

Anschließend erfolgt gemäß der Reaktionsstufe c) die Reduktion der Nitrogruppen in die entsprechenden aromatisch gebundenen Aminogruppen. Vorzugsweise erfolgt diese Reduktion durch katalytische Hydrierung beispielsweise unter Verwendung von Raney-Nickel oder Palladium als Katalysator. Die Hydrierung erfolgt im allgemeinen in alkoholischer Lösung, wobei als Lösungsmittel beispielsweise Methanol, Ethanol oder Isopropanol zum Einsatz gelangt. Die zu hydrierenden Nitroverbindungen werden hierbei im allgemeinen in Form einer 10 bis 50 Gew.-%igen Lösung eingesetzt. Die Hydrierung erfolgt, gegebenenfalls unter Druck, bei einer Temperatur von 20 bis 150 °C, vorzugsweise 30 bis 110 °C. Die Überführung der Nitrogruppen in die entsprechenden Aminogruppen kann selbstverständlich auch nach der an sich bekannten Reduktionsmethode unter Verwendung von beispielsweise Eisen, Zink oder Zinn als Reduktionsmittel erfolgen. Im Anschluß an die Überführung der Nitrogruppen in die entsprechenden Aminogruppen wird der Katalysator beispielsweise durch Filtration entfernt und das Lösungsmittel abdestilliert. Der Gehalt des beim Reaktionsschritt c) anfallenden Polyamingemischs an trifunktionellen Polyaminen kann gewünschtenfalls vor dem Reaktionsschritt d) durch destillative Abtrennung von niedriger oder höher siedenden Bestandteilen erhöht werden.

Die so gegebenenfalls destillativ aufgearbeiteten Polyamine bzw. Polyamingemische werden anschließend gemäß Reaktionsschritt d) in an sich bekannter Weise durch Phosgenierung in die entsprechenden Polyisocyanate überführt. Als Lösungsmittel dient hierbei beispielsweise Chlorbenzol, Dichlorbenzol oder Toluol. Die erfindungsgemäßen Verfahrensprodukte liegen schließlich nach Abdestillieren des Hilfslösungsmittels als Rückstand vor. Auch auf dieser Stufe kann der Gehalt der Verfahrensprodukte an Triisocyanaten durch destillative Abtrennung von niedriger oder höher als die Triisocyanate siedenden Bestandteilen erhöht werden.

Das erste Verfahren zur Herstellung der erfindungsgemäßen Polyisocyanatgemische gestattet die Herstellung von erfindungsgemäßen Polyisocyanatgemischen, wobei die Funktionalität der letztendlich erhaltenen Verfahrensprodukte durch den Nitrierungsgrad bei der Reaktionsstufe b) und/oder durch die gegebenenfalls im Anschluß an die Reaktionsstufen c) oder d) erfolgende destillative Aufarbeitung, und dies gilt im Prinzip auch für die nachstehend beschriebenen Verfahren, innerhalb des o. g. Bereichs eingestellt werden kann.

Wie dargelegt, ist eine Einstellung der Funktionalität der erfindungsgemäßen Polyisocyanatgemische sowohl durch den Nitrierungsgrad als auch durch die im Anschluß an die Reaktionsschritte c) bzw. d) erfolgende destillative Aufarbeitung möglich. Hierbei ist jedoch von besonderem Interesse daß oftmals alkylsubstituierte erfindungsgemäße Polyisocyanatgemische bei gleichner NCO-Funktionalität und gleicher Konstitution der eingesetzten Ausgangsmaterialien unterschiedliche Eigenschaften aufweisen können, je nach dem, ob die NCO-Funktionalität durch den Nitrierungsgrad oder destillativ eingestellt worden ist. Dieser Effekt wird insbesondere dann beobachtet, wenn bereits auf der Dinitro-Stufe mehr als ein Isomeres vorliegt bzw. durchlaufen wird, d. h. der Effekt tritt nicht auf, falls beim ersten Verfahren in der Reaktionsstufe a) 1-Alkyl-4-nitro-benzol eingesetzt wird, da in diesen Fällen praktisch nur ein Isomeres auf der Dinitro-Stufe vorliegt. Der Effekt ist jedoch dann zu beobachten, wenn beim ersten

Verfahren als Reaktionspartner für das 4-Nitrobenzylhalogenid 1-Alkyl-2-nitro-benzol zum Einsatz gelangt, da dann auf der Dinitro-Stufe 2 Isomere vorliegen. Dieser auch bei den nachfolgend beschriebenen 2. bis 4. Verfahren zur Herstellung der erfindungsgemäßen Gemische zu beobachtende Effekt ist damit zu erklären, daß unterschiedliche Dinitro-Isomere im allgemeinen auch eine unterschiedliche Reaktivität bei der Weiternitrierung zeigen, und sich demzufolge mit fortschreitender Nitrierung das Spektrum der verbleibenden Dinitroverbindungen in seiner Zusammensetzung kontinuierlich verändert.

Das zweite Verfahren zur Herstellung von erfindungsgemäßen Polyisocyanatgemischen ist dadurch gekennzeichnet, daß man

a) ein 4-Nitrobenzylhalogenid in Gegenwart von Friedel-Crafts-Katalysatoren mit Alkylbenzolen zu einem Gemisch von Mononitroverbindungen der Formel

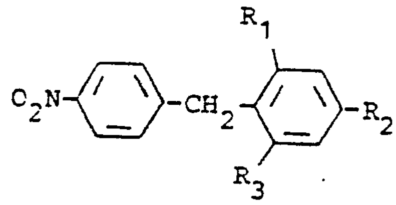

umsetzt, das Umsetzungsprodukt vom Katalysator befreit,

b) das gemäß a) erhaltene Umsetzungsprodukt einer Nitrierungsreaktion unter Bildung von Polynitroverbindungen der Formel

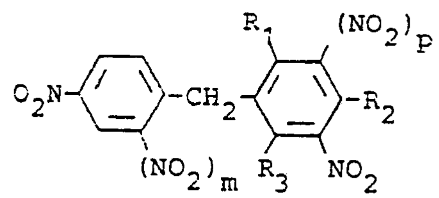

unterzieht, wobei $R_1$, $R_2$, $R_3$ m und p die bei der Schilderung der Stufe b) des 1. Verfahrens genannte Bedeutung haben,

c) die gemäß b) erhaltenen Polynitroverbindungen durch Hydrierung bzw. Reduktion der Aminogruppen in die entsprechenden aromatischen Polyaminverbindungen überführt und

d) die gemäß c) erhaltenen Polyaminoverbindungen durch Phosgenierung in die entsprechenden Polyisocyanate überführt,

wobei man gegebenenfalls

e) den Gehalt der gemäß c) erhaltenen Polyamingemische an Triamino-Isomeren vor der Phosgenierungsreaktion und/oder den Gehalt der gemäß d) erhaltenen Polyisocyanatgemische an dem entsprechenden Triisocyanato-Isomeren durch destillative Abtrennung von niedriger bzw. höher siedenden Nebenprodukten erhöht.

Die Stufe a) des zweiten Verfahrens entspricht mit der Ausnahme der Verwendung von Alkylbenzol als Ausgangsmaterial der Stufe a) des ersten erfindungsgemäßen Verfahrens, wobei jedoch im allgemeinen die Siedetemperatur des hier in einem entsprechenden Überschuß eingesetzten Alkylbenzols die obere Grenze des Temperaturbereichs darstellt. Das gemäß a) erhaltene, vom Katalysator befreite Kondensationsprodukt wird anschließend in der Stufe b) in an sich bekannter Weise zu einem Gemisch isomerer Polynitroverbindungen der genannten allgemeinen Formel nitriert. Diese Nitrierung erfolgt beispielsweise in Gegenwart eines geeigneten Lösungsmittels wie Methylenchlorid unter Verwendung von « Nitriersäure », d. h. ein Gemisch aus konzentrierter Schwefelsäure und Salpetersäure, vorzugsweise hochkonzentrierter, ca. 98 %iger Salpetersäure. Die Menge der Nitriersäure wird so bemessen, daß für jedes Mol an gemäß a) erhaltener Mononitroverbindung ca. 1,1 bis 3,1, vorzugsweise 1,2 bis 2,2 Mol Salpetersäure zur Verfügung stehen, so daß im Nitrierungsprodukt im statistischen Mittel mehr als 2, vorzugsweise 2,1 bis 3 Nitrogruppen pro Molekül vorliegen. Die Nitrierung wird im allgemeinen im Temperaturbereich zwischen – 20 und 100 °C, vorzugsweise 0 bis 70 °C, ein- oder zweistufig durchgeführt. Anschließend wird die nach der Nitrierungsreaktion vorliegende organische Phase von der Säure durch Phasentrennung, Auswaschen mit Wasser und beispielsweise Natriumcarbonatlösung befreit. Schießlich erfolgt die destillative Entfernung des Hilfslösungsmittels gegebenenfalls unter anschließendem Austreiben von Lösungsmittelresten mittels Wasserdampf.

Die weitere Umsetzung der so erhaltenen Polynitroverbindungen erfolgt in völliger Analogie zu den Reaktionsschritten c) und d) des ersten erfindungsgemäßen Verfahrens.

Beim zweiten erfindungsgemäßen Verfahren entstehen als erfindungsgemäße Polyisocyanatgemische im allgemeinen Gemische, die ca. 70 bis 90 Gew.-% an Di- und Polyisocyanato-2-, -4- oder -6-alkyldiphenylmethan und 10 bis 30 Gew.-% an anderen, analytisch nicht näher charakterisierten Alkyl-

substituierten Polyisocyanato-diphenylmethan-Isomeren enthalten. Die Polyisocyanate entsprechen somit in erster Näherung der Formel

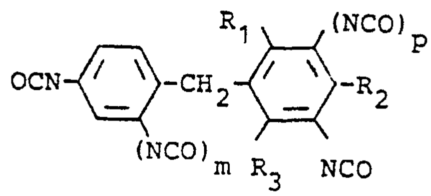

wobei

$R_1$, $R_2$, $R_3$, m und p die obengenannte Bedeutung haben.

Beim dritten und vierten Verfahren zur Herstellung der erfindungsgemäßen Polyisocyanatgemische liegen, im Unterschied zum 1. und 2. erfindungsgemäßen Verfahren, in den als Verfahrensprodukt anfallenden Gemischen als weitere Triisocyanat-Isomeren auch gegebenenfalls alkylsubstituierte 3,5,2'-Triisocyanato-diphenylmethane vor.

Das dritte Verfahren zur Herstellung der erfindungsgemäßen Polyisocyanatgemische ist dadurch gekennzeichnet, daß man

a) technische, durch Nitrierung von Benzylchlorid erhaltene, 10 bis 50 Gew.-%, bezogen auf Gesamtgemisch, 2-Nitrobenzylchlorid und 50 bis 90 Gew.-%, gezogen auf Gesamtgemisch, 4-Nitrobenzylchlorid neben untergeordneten Mengen, d. h. bis maximal 20 Gew.-%, vorzugsweise bis maximal 15 Gew.-% an 3-Nitrobenzylchlorid enthaltende Nitrobenzylchlorid-Isomerengemische in Gegenwart von Friedel-Crafts-Katalysatoren mit Alkylbenzol mit zu einem im wesentlichen Mononitroverbindungen der Formeln

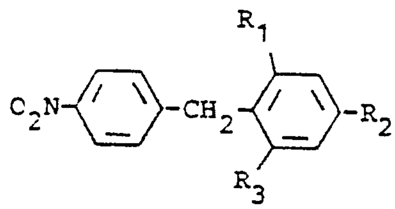

und

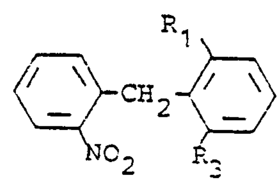

enthaltenden Friedel-Crafts-Kondensat umsetzt, den Katalysator aus dem Kondensat entfernt,

b) das gemäß a) erhaltene Kondensat einer Nitrierungsreaktion unter Bildung von im wesentlichen Nitroverbindungen der Formel

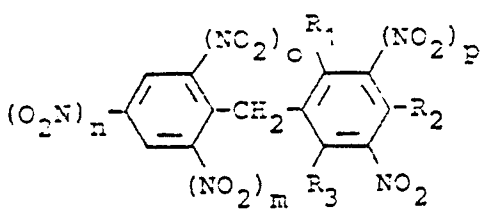

enthaltenden Gemisch aromatischer Polynitroverbindungen unterzieht, in denen jedes der beiden aromatischen Ringe mindestens eine Nitrogruppe aufweist und wobei $R_1$, $R_2$, $R_3$, m, n, o und p die obengenannte Bedeutung haben,

c) die gemäß b) erhaltenen Nitroverbindungen durch Hydrierung bzw. Reduktion der Nitrogruppen in die entsprechenden aromatischen Polyamine überführt,

d) die gemäß c) erhaltenen Polyamine durch Phosgenierung in die entsprechenden Polyisocyanate überführt,

wobei man gegebenenfalls

e) den Gehalt der gemäß c) erhaltenen Polyamingemische an Triamino-Isomeren vor der Phosgenierungsreaktion und/oder den Gehalt der gemäß d) erhaltenen Polyisocyanatgemische an dem entsprechenden Triisocyanato-Isomeren durch destillative Abtrennung von niedriger bzw. höher siedenden Nebenprodukten erhöht.

Die Durchführung des dritten Verfahrens erfolgt in völliger Analogie zum zweiten Verfahren, wobei man jedoch anstelle von 4-Nitro-benzylhalogenid als Ausgangsmaterial ein technisches Nitrobenzylchlorid-Isomerengemisch verwendet, welches beispielsweise 10 bis 50 Gew.-%, vorzugsweise 30 bis 40 Gew.-%, 2-Nitrobenzylchlorid und 50 bis 90 Gew.-%, vorzugsweise 50 bis 60 Gew.-%, 4-Nitrobenzylchlorid neben untergeordneten Mengen an 3-Nitrobenzylchlorid enthält. Unter « Untergeordneten Mengen » sind hierbei, bezogen auf Gesamtgewicht, maximal 20 Gew.-%, vorzugsweise maximal 15 Gew.-%, zu verstehen.

Beim dritten erfindungsgemäßen Verfahren entstehen als Verfahrensprodukte in erster Linie, d. h. zu mindestens 60, vorzugsweise zu mindestens 70 Gew.-%, bezogen auf Gesamtgemisch, an Polyisocyanaten, die der allgemeinen Formel

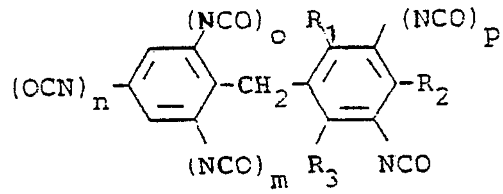

entsprechen, in denen jeder der beiden aromatischen Ringe mindestens eine Isocyanatgruppe aufweist und wobei die Werte von m, n, o und p innerhalb der angegebenen Grenzen, wie bereits dargelegt, durch den Nitrierungsgrad und die evtl. destillative Aufarbeitung auf der Amin- bzw. Isocyanatstufe eingestellt werden können.

Das vierte Verfahren zur Herstellung der erfindungsgemäßen Polyisocyanatgemische ist dadurch gekennzeichnet, daß man

a) ein Benzylhalogenid in Gegenwart von Friedel-Crafts-Katalysatoren oder Benzylalkohol in Gegenwart von Säurekatalysatoren mit Alkylbenzolen mit 1 bis 2 Kohlenstoffatomen im Alkylrest zu einem im wesentlichen aus Kohlenwasserstoffen der Formel

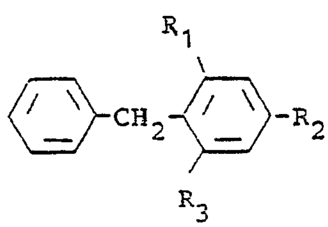

bestehenden Kondensat umsetzt, wobei einer der Reste $R_1$, $R_2$ oder $R_3$ für eine Alkylgruppe und die beiden anderen für Wasserstoff stehen,

b) das aus dem a) erhaltene Kondensat destillativ in reiner Form erhaltene Alkyl-diphenylmethan-Isomerengemisch der zuletzt genannten Formel einer Nitrierung unterzieht, so daß im statistischen Mittel je Kohlenwasserstoffmolekül mehr als 2 Nitrogruppen eingeführt werden,

c) die gemäß b) erhaltenen Polynitroverbindungen durch Hydrierung bzw. Reduktion der Nitrogruppen in die entsprechenden Polyamine überführt,

d) die gemäß c) erhaltenen Polyamine durch Phosgenierung in die entsprechenden Polyisocyanate überführt,

wobei man gegebenenfalls

e) den Gehalt der gemäß c) erhaltenen Polyamingemische an Triamino-Isomeren vor der Phosgenierungsreaktion und/oder den Gehalt der gemäß d) erhaltenen Polyisocyanatgemische an dem entsprechenden Triisocyanato-Isomeren durch destillative Abtrennung von niedriger bzw. höher siedenden Nebenprodukten erhöht.

Bei der Stufe a) des vierten Verfahrens wird anstelle eines Nitrobenzylhalogenids das unsubstituierte Benzylhalogenid oder benzylalkohol eingesetzt.

Im Falle der Verwendung eines Benzylhalogenids gelten bezüglich der Durchführung der Stufe a) insbesondere bezüglich der Mengenverhältnisse der Reaktionspartner die bei der Schilderung des ersten und zweiten Verfahrens gemachten Ausführungen. Vorzugsweise wird jedoch bei einem Molverhältnis von Alkylbenzol zu Benzylchlorid von 5 : 1 bis 20 : 1 gearbeitet, besonders bevorzugt ist 8 : 1 bis 15 : 1. Die Kondensationsreaktion kann jedoch im Extremfall in der Gasphase bei Temperaturen von bis zu 300 °C durchgeführt werden. Die bevorzugte Temperatur zur Durchführung der Stufe a) liegt jedoch innerhalb der für die Stufe a) des zweiten Verfahrens gültigen Bereiche.

Im Falle der Verwendung von Benzylalkohol als Ausgangsmaterial werden als Katalysatoren

schwerflüchtige starke Säuren eingesetzt, wie z. B. Schwefelsäure, Aryl- und Alkylsulfonsäuren, Phosphorsäure oder beispielsweise Sulfonsäuregruppen aufweisende Festbettkatalysatoren wie Sulfonsäuregruppen aufweisende Ionenaustauscher oder anorganische saure Zentren aufweisende Feststoffkatalysatoren (Tonsile, Zeolithe etc.).

Bezüglich der Mengenverhältnisse der Reaktionspartner gelten die für die Reaktion zwischen Benzylchlorid und Alkylbenzol gemachten Ausführungen sinngemäß, d. h. auch hier wird das Alkylbenzol in einem diesen Ausführungen entsprechenden Überschuß eingesetzt. Die Reaktionstemperatur liegt hier im allgemeinen zwischen − 20 und 300 °C, vorzugsweise 20 bis 110 °C. Das anfallende Kondensat wird beispielsweise durch Auswaschen mit Wasser bei homogener Katalyse, bzw. durch Filtrieren bei heterogener Katalyse, vom Katalysator und durch Abdestillieren vom überschüssigen Alkylbenzol befreit und in destillativ von geringen Mengen an höhermolekularen Kondensaten befreiter Form der Stufe b) zugeführt.

Bei dieser Reaktionsstufe b) wird das Kondensat einer Nitrierung unterzogen, wobei im Prinzip die bei der Schilderung der Stufe b) des zweiten Verfahrens gemachten Ausführungen gelten, jedoch mit dem Unterschied, daß hier für jedes Mol an zu nitrierendem Kohlenwasserstoff eine solche Menge an Nitriersäure zur Verfügung steht, daß auf jedes Mol an zu nitrierendem Kohlenwasserstoff mehr als 2 Mol Salpetersäure zur Verfügung stehen, so daß als Nitrierungsprodukte Gemische entstehen, die im statistischen Mittel pro Molekül mehr als 2 Nitrogruppen enthalten. Die Nitrierungsreaktion kann ein- oder zweistufig durchgeführt werden. Dies bedeutet, daß die Nitrier säure dem Reaktionsgemisch portionsweise zugeführt werden kann. Im Falle einer zweistufigen Nitrierung ist zweckmäßig die in der 2. Stufe zur Einführung der 2. und weiterer Nitrogruppen anfallende Restsäure nach erneuter Zugabe von Salpetersäure bei der 1. Stufe erneut einzusetzen.

Die weitere Verarbeitung der so erhaltenen Alkyl-substituierten Dinitrodiphenylmethan-Isomeren erfolgt in völliger Analogie zu der bereits in Zusammenhang mit dem ersten oder zweiten Verfahren gemachten Ausführungen.

Beim vierten Verfahren werden im allgemeinen im Gemisch mit bis zu 30 Gew.-%, bezogen auf Gesamtgemisch, an anderen, analytisch nicht genau erfaßbaren Alkyl-substituierten Polyisocyanatodiphenylmethan-Isomeren vorliegende Polyisocyanate der Formel

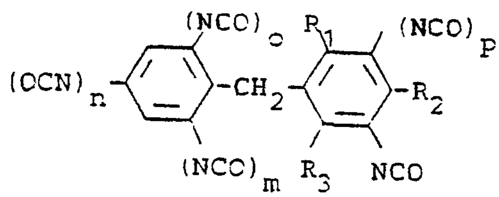

erhalten, in denen jeder der beiden aromatischen Ringe mindestens eine Isocyanatgruppe aufweist und wobei einer der Reste $R_1$, $R_2$ oder $R_3$ für einen Alkylrest und die beiden anderen der genannten Reste für Wasserstoffatome stehen und in welcher m, n, o und p die bereits genannte Bedeutung haben.

Die Verfahren zur Herstellung der erfindungsgemäßen Polyisocyanatgemische ermöglichen die Herstellung neuartiger Polyisocyanatgemische der Diphenylmethanreihe, deren NCO-Funktionalität innerhalb eines weiten Bereichs dem jeweiligen Verwendungszweck angepaßt werden kann. Diese Einstellung der NCO-Funktionalität kann sowohl durch den Nitrierungsgrad als auch durch die destillative Aufarbeitung auf der Amin- bzw. Isocyanatstufe erfolgen. Wie bereits bei der Schilderung des ersten Verfahrens ausgeführt, können hierbei Polyisocyanate gleicher Funktionalität jedoch unterschiedlicher Reaktivität hergestellt werden, so daß hierin eine weitere Möglichkeit gesehen werden kann, die erfindungsgemäßen Polyisocyanatgemische dem jeweiligen Vewendungszweck in optimaler Weise anzupassen. Die Verfahren gestatten die Herstellung von Polyisocyanatgemischen, die eine mittlere NCO-Funktionalität von größer als 2 aufweisen, ohne daß hierbei das mittlere Molekulargewicht der Polyisocyanate, wie dies bei den klassischen MDI-Produkten der Fall ist, wesentlich erhöht werden müßte. Die erfindungsgemäßen Gemische weisen mittlere NCO-Funktionalität von 2,2 bis 2,7 auf, so daß die Summe aus m + n + o + p in der allgemeinen Formel für die erfindungsgemäßen Gemische in Mittel 1,2 bis 1,7 liegt. Die erfindungsgemäßen Polyisocyanatgemische weisen eine wesentlich niedrigere Viskosität als Phosgenierungsprodukte von Anilin/Formaldehyd-Kondensaten einer gleichen mittleren NCO-Funktionalität auf. Außerdem unterscheiden sich die erfindungsgemäßen Polyisocyanate bzw. Polyisocyanatgemische von den Phosgenierungsprodukten von Anilin/Formaldehyd-Kondensaten durch ihre wesentlich verringerte Tendenz, bei erniedrigten Temperaturen teilweise zu kristallisieren. Die Eigenschaften der erfindungsgemäßen Produkte insbesondere bezüglich ihrer Eignung als Ausgangsmaterial zur Herstellung von Polyurethanen sind von der Stellung des Alkylsubstituenten bzw. von dem jeweiligen Anteil der jeweiligen, sich durch die Stellung des Alkylsubstituenten unterscheidenden Isomeren völlig unabhängig. Die erfindungsgemäßen Gemische stellen technische Gemische dar, die, wie dargelegt, oftmals untergeordnete Mengen, d. h. bis zu 40 Gew.-% an analytisch, d. h. insbesondere gaschromatographisch nicht eindeutig charakterisierbaren Bestandteilen enthalten. Es handelt sich hierbei im

allgemeinen um gegebenenfalls einen Alkylsubstituenten aufweisende Polyisocyanato-Diphenylmethan-Isomere, in denen 2 der NCO-Gruppen in 2,4'-, 4,4'- oder 3,3'-Stellung angeordnet sind, bzw. um Gemische derartiger Isomerer. Der Gehalt der erfindungsgemäßen Gemische an diesen Nebenprodukten in den angegebenen Grenzen beeinträchtigt jedoch in keiner Weise deren vorteilhafte Eigenschaften. Allen erfindungsgemäßen Polyisocyanatgemischen gemeinsam sind die genannten Vorteile im Vergleich zu den klassischen Phosgenierungsprodukten von Anilin/Formaldehyd-Kondensaten. Die erfindungsgemäßen Polyisocyanatgemische insbesondere die erfindungsgemäß bevorzugten Alkyl-substituierten Verbindungen bzw. deren Gemische stellen besonders wertvolle neuartige Ausgangsmaterialien zur Herstellung von Polyurethankunststoffen dar. Die neuen erfindungsgemäßen Polyisocyanatgemische können bei allen bislang bekannt gewordenen Verfahren zur Polyurethankunststoffherstellung unter Verwendung von Toluylendiisocyanaten (TDI) und/oder MDI anstelle dieser Polyisocyanate des Standes der Technik oder im Gemisch mit diesen eingesetzt werden.

Beispiel 1 (1. Verfahren)

a) Bei Raumtemperatur wurden 171,6 g (1 Mol) 4-Nitrobenzylchlorid in 755 g (5 Mol) 1-Ethyl-2-nitrobenzol gelöst und mit 16,2 g (0,1 Mol) wasserfreiem Eisen-III-chlorid versetzt.

Unter Rühren wurde das Reaktionsgemisch langsam aufgeheizt auf 100-110 °C und bis zum Nachlassen der Chlorwasserstoffentwicklung bei dieser Temperatur gehalten. Nach ca. 5 h war die Reaktion beendet.

Nach dem Abkühlen auf Raumtemperatur wurde mit 500 ml Methylenchlorid versetzt und einmal mit 200 ml halbkonzentrierter Salzsäure und dreimal mit je 500 ml Wasser gewaschen. Nach dem Trocknen wurde zunächst das Methylenchlorid und dann das überschüssige 1-Ethyl-2-nitro-benzol abgetrennt.

Der Rückstand (274 g) enthieht in seinem verdampfbaren Anteil gemäß GC :

2 Gew.-% 1-Ethyl-2-nitro-benzol
3,8 Gew.-% nicht identifizierte Dinitroverbindung
28,5 Gew.-% 3,4'-Dinitro-2-ethyldiphenylmethan
67,7 Gew.-% 3,4'-Dinitro-4-ethyldiphenylmethan

b) 200 g (ca. 0,7 Mol) des Rohgemisches von a) wurden in 400 ml Dichlormethan aufgenommen, wozu bei 20 °C unter Rühren langsam Nitriersäure zugegeben wurde bestehend aus einer Mischung von

65 g (1,0 Mol) 98 %iger $HNO_3$ und
100 g (1,0 Mol) 98 %iger $H_2SO_4$.

Nach beendeter Zugabe wurde noch ca. 2 h bei 28-30 °C nachgerührt und dann die Mischsäure abgetrennt.

Die verbleibende organische Phase wurde zweimal mit je 200 ml Wasser, einmal mit 2 %iger Natriumcarbonatlösung und noch zweimal mit Wasser gewaschen. Anschließend wurde das Methylenchlorid bei Normaldruck bis 90 °C Sumpftemperatur abdestilliert, die Lösungsmittelreste wurden mit Wasserdampf ausgetrieben. Das resultierende Produkt (220 g) wurde im Wasserstrahlvakuum bei 90 °C/16 mbar entwässert.

c) 200 g des Rohproduktes von b) wurden in 600 ml Tetrahydrofuran aufgenommen, mit 30 g frischem Raney-nickel versetzt und bei 50 °C mit Wasserstoff bei 100 bar bis zur Druckkonstanz reduziert.

Nach Beendigung der exotermen Reaktion wurde noch 1 h bei 80 °C und 100 bar Wasserstoffdruck gehalten. Danach wurde entspannt, der Katalysator abfiltriert und Lösungsmittel und Wasser abdestilliert.

Der Rückstand (145 g) wurde an der Ölpumpe bei 0,1333 mbar (0,1 Torr) destilliert :

Vorlauf (11 g) (80-140 °C) :
niedrig siedende Verbindungen überwiegend Diamino-ethylbenzol
Hauptfraktion (123 g) (140-280 °C) :
0,4 Gew.-% nicht identifizierte Diaminoverbindung
4,6 Gew.-% 2-Ethyl-3,4'-diaminodiphenylmethan
22,6 Gew.-% 4-Ethyl-3,4'-diaminodiphenylmethan
20,2 Gew.-% 2-Ethyl-3,5,4'-triaminodiphenylmethan und 4-Ethyl-3,2,4'-triaminodiphenylmethan (nicht aufgetrennt)
43,8 Gew.-% 4-Ethyl-3,5,4'-triaminodiphenylmethan
8,4 Gew.-% höhersiedende Polyarylamine
Rückstand (ca. 10 g)

d) Das Destillat von c) (122 g) wurde in 1,25 l Monochlorbenzol gelöst und bei 0 °C unter Rühren und Kühlen zu einer Lösung von 500 g Phosgen in 1 l Monochlorbenzol langsam zugetropft. Anschließend wurde unter weiterem Durchleiten von Phosgen das Reaktionsgemisch langsam aufgeheizt und eine Stunde am Rückfluß gekocht.

Im Wasserstrahlvakuum wurde anschließend das Monochlorbenzol abgezogen und bei weiter reduziertem Druck (0,53 mbar (0,4 Torr)) das Isocyanat destilliert. Nach einem Vorlauf von ca. 15 g (70-180 °C) gingen bei 180-210 °C 113 g über. Als Destillationsrückstand verblieben 38 g. Das Destillat (Hauptfraktion) bestand nach GC-Analyse zu

22,3 Gew.-% aus 2-Kerndiisocyanaten und
77,2 Gew.-% aus 2 Kerntriisocyanaten

und wurde anschließend nochmals fraktioniert feindestilliert.
1. Fraktion : 49 g (160-175 °C/0,266 6 mbar (0,2 Torr))

34,9 Gew.-% 2-Kerndiisocyanat
61,1 Gew.-% 2-Kerntriisocyanat

2. Fraktion : 58 g (175-178 °C/0,266 6 mbar (0,2 Torr)) einer farblosen Flüssigkeit (NCO-Gehalt 39,4 Gew.-%) die aus einem Gemisch aus 2-Ethyl-3,5,4'-triisocyanatodiphenylmethan und 4-Ethyl-3,5,4'-triisocyanatodiphenylmethan besteht.

Beispiel 2 (2. Verfahren)

a) 343 g (2 Mol) 4-Nitrobenzylchlorid wurden in 920 g (10 Mol) trockenem Toluol gelöst und mit 32,4 g (0,2 Mol) wasserfreiem Eisen-III-chlorid versetzt.

Unter Rühren wurde langsam bis auf 80 °C aufgeheizt, 5 Stunden bei dieser Temperatur gehalten, nach beendeter Chlorwasserstoffentwicklung 1 Stunde am Rückfluß gekocht, nach dem Abkühlen in Eiswasser gegossen und dreimal mit je 500 ml Wasser säurefrei gewaschen.

Nach dem Trocknen und Abdestillieren des überschüssigen Toluols bei Normaldruck wurde der Rückstand (438 g) bei vermindertem Druck (0,666 5 mbar (0,5 Torr)) destilliert. Zwischen 140 und 150 °C gingen 398,4 g (87,8 % der Theorie) eines niederviskosen gelben Öles über, einem Gemisch aus 2-, 3- und 4-Methyl-4'-nitrodiphenylmethan, welches gaschromatographisch nicht mehr aufgetrennt werden konnte.

b) 45,5 g (0,2 Mol) des Destillats von a) wurden zusammen mit 100 g 83 %iger Schwefelsäure bei 20-25 °C vorgelegt. Unter Rühren und Kühlen wurden bei dieser Temperatur 38 ml einer Mischsäure, (0,4 Mol), bestehend aus 40 g 98 %iger Schwefelsäure und 25,5 g 98 %iger Salpetersäure zugetropft. Nach beendeter Zugabe wurde noch 20 Minuten bei 20-25 °C nachgerührt, anschließend die Schwefelsäure abgetrennt und das Nitroprodukt mit 150 ml Wasser versetzt, 1 Stunde am Rückfluß erhitzt und nach dem Abkühlen auf 80-90 °C in 100 ml Toluol aufgenommen. Nach dem Abtrennen der wäßrigen Phase wurde die organische Phase nacheinander zweimal mit 3 %iger wäßriger Soda (je 50 ml) und zweimal Wasser (je 50 ml) gewaschen. Danach wurde unter reduziertem Druck das Toluol bis 90 °C Sumpftemperatur abdestilliert. Als Rückstand berblieben 54 g.

c) 52 g des Rückstandes von b) wurden analog Beispiel 1b) in die Aminverbindungen übergeführt und ergaben 37,7 g Rohamin, die an der Ölpumpe destilliert wurden. Nach einem Vorlauf von ca. 3 ml wurden bei einem Druck von 0,133 mbar (0,1 Torr) und einer Destillationstemperatur von 180 bis 245 °C 30 g Destillat erhalten, die nach GC folgende Zusammensetzung aufwiesen :

1,5 Gew.-% niedrigsiedende Amine
64,9 Gew.-% Diamino-methyldiphenylmethane
33,4 Gew.-% Triamino-methyldiphenylmethane

Die Diaminofraktion enthielt

93 Gew.-% 2-, 4- und 6-Methyl-3,4'-diamino-diphenylmethane
7 Gew.-% andere Isomere

d) 29 g destilliertes Amingemisch von c) wurden in 250 ml Monochlorbenzol gelöst und bei 0 °C unter Rühren und Kühlen zu einer Lösung von 100 g Phosgen in 250 ml Monochlorbenzol langsam zugetropft, unter weiterem Durchleiten von Phosgen aufgeheizt und drei Stunden unter Rückfluß nachphosgeniert. Anschließend wurde das Phosgen ausgeblasen und das Lösungsmittel eingeengt. Der Rückstand (Rohisocyanat) wurde viermal mit je 150 ml n-Hexan extrahiert, die vereinigten Extrakte gemeinsam eingeengt und der Rückstand (34 g) destilliert. Nach einem Vorlauf von 2,5 g gingen bei einem Druck von 0,133 3 mbar (0,1 Torr) und einer Temperatur von 158-200 °C 28,5 g einer Hauptfraktion über, die bei einem NCO-Gehalt von 34,5 % aus

73 Gew.-% Diisocyanatomethyldiphenylmethanen
27 Gew.-% Triisocyanatomethyldiphenylmethanen

bestand.

Beispiel 3 (2. Verfahren)

a) 171,5 g (1 Mol) 4-Nitrobenzylchlorid wurden in 263 g (2,5 Mol) trockenem Ethylbenzol gelöst und bei 40 °C unter Rühren langsam zu 3 g wasserfreiem Eisen-III-chlorid in 800 g (7,5 Mol) trockenem Ethylbenzol zugetropft.

Unter Rühren wurde langsam bis auf 80 °C aufgeheizt, und bei dieser Temperatur gehalten. Nach beendeter Chlorwasserstoffentwicklung wurde 2 Stunden bei 70 bis 80 °C nachgerührt und nach dem Abkühlen dreimal mit je 500 ml Wasser säurefrei gewaschen.

Nach dem Trocknen und Abdestillieren des überschüssigen Ethylbenzols wurde der Rückstand (238 g) bei vermindertem (Druck (0,133 3 mbar (0,1 Torr)) destilliert. Zwischen 135 und 145 °C gingen 227 g (94,2 % der Theorie) eines niederviskosen hellgelben Öles über, es verblieben 6 g Rückstand.

In einem Parallelansatz mit einem Ethylenbenzolüberschuß von 5 : 1 betrug die Ausbeute 219 g (91 % der Theorie) an destilliertem Produkt praktisch gleicher Zusammensetzung.

Das GC-Spektrum zeigte :

ca. 2 Gew.-% niedrigsiedende Verbindung
55 Gew.-% 2- (und 3-) Ethyl-4'-nitrodiphenylmethan
45 Gew.-% 4-Ethyl-4'-nitrodiphenylmethan

b) 100 g 83 %iger Schwefelsäure wurde bei 20-30 °C vorgelegt. Dazu wurden unter Rühren und Kühlen gleichzeitig und in konstantem stöchiometrischen Verhältnis von 2 : 1 zugetropft :

98,6 g (0,6 Mol) einer Mischsäure bestehend aus 60 g 98 %iger Schwefelsäure und 38,6 g 98 %iger Salpetersäure und

72,5 g (0,3 Mol) des destillierten Isomerengemisches der 4'-Nitro-ethyldiphenylmethane von a).

Nach beendeter Zugabe wurde noch 1 Stunde bei 30-35 °C nachgerührt. Zur weiteren Aufarbeitung wird die Schwefelsäurephase abgetrennt und die organische Phase zweimal mit je 250 ml und einmal mit 250 ml 2 %iger Sodalösung und zweimal mit je 250 ml Wasser gewaschen. Bei Normaldruck wurden ca. 500 ml Lösungsmittel abdestilliert und der Rückstand auf ca. 0 °C abgekühlt. Die ausgefallenen Kristalle werden abgesaugt. Ausbeute : 78 g Rohprodukt.

c) 75 g des Rohproduktes von b) wurden analog Beispiel 1 b) in die Aminoverbindungen übergeführt und ergaben 59,5 g Rohamin, welches gemäß Gaschromatogramm die folgende Zusammensetzung im verdampfbaren Anteil aufwies :

7 Gew.-% niedrigsiedende Amine
31 Gew.-% Diamino-ethyldiphenylmethane
62 Gew.-% Triamino-ethyldiphenylmethane

d) 56 g des Rohprodukts von c) wurden in 200 ml Methylenchlorid gelöst und bei 0 °C unter Rühren und Kühlen zu einer Lösung von 200 g Phosgen in 1 000 ml Monochlorbenzol langsam zugetropft. Unter weiterem Durchleiten von Phosgen wurde aufgeheizt, wobei das Methylenchlorid abdestillierte, bis zum Sieden des Monochlorbenzols. Nachdem in diesem Stadium von 3 Stunden unter Durchleiten von Phosgen nachphosgeniert worden war, wurde das Phosgen ausgeblasen und das Lösungsmittel eingeengt. Der verbleibende Rückstand (Rohphosgenat) wurde viermal mit je 250 ml n-Hexan heiß extrahiert.

Aus den vereinigten Extrakten wurde druch Einengen ein Isocyanatrückstand von 55 g erhalten, der an der Ölpumpe bei 0,133 3-0,4 mbar (0,1-0,3 Torr) destilliert wurde und neben 7 g Vorlauf (der schon Isocyanat enthielt) und 6 g Rückstand zwischen 185 und 205 °C eine Hauptfraktion (42 g) ergab mit einem NCO-Gehalt von 36,5 NCO (bezogen auf MG 42) und der Zusammensetzung (GC) :

ca. 34 Gew.-% Diisocyanato-ethyldiphenylmethanen
ca. 66 Gew.-% Triisocyanato-ethyldiphenylmethanen.

Beispiel 4 (3. Verfahren)

a) 254 g (2 Mol) Benzylchlorid wurden in 400 ml Methylenchlorid zusammen mit 295 g (3 Mol) 98 %iger Schwefelsäure bei − 5 °C vorgelegt. Unter Rühren und Kühlen wurden dazu bei − 5 °C bis 0 °C im Verlauf von 1,5 Stunden 190 g (3 Mol) 100 %ige Salpetersäure zugetropft. Nach beendeter Zugabe wurde noch eine Stunde bei 0 °C nachgerührt.

Das Nitriergemisch wurde anschließend in 1,2 l Eiswasser eingerührt, die organische Phase abgetrennt, zweimal mit je 500 ml Wasser gewaschen, mit 300 ml gesättigter Natriumbicarbonatlösung geschüttelt, noch einmal mit Wasser gewaschen und über Natriumsulfat getrocknet.

Bei Normaldruck wurde das Methylenchlorid abgezogen. Der Rückstand wurde bei vermindertem Druck (0,133 3 mbar (0,1 Torr)) destilliert. Dabei gingen zwischen 80 °C und 120 °C Kopftemperatur 328 g

eines Gemisches der Mononitrobenzylchloride über. Der Destillationsrückstand betrug 6 g, so daß bei weiteren Ansätzen auf eine Destillation verzichtet wurde.

257,3 g (1,5 Mol) des Nitrobenzylchlorid-Isomerengemisches wurde in 690 g (7,5 Mol) trockenem Toluol aufgenommen. Unter Rühren wurde dazu bei Raumtemperatur im Verlaufe von 2 Stunden eine Lösung von 31 g (0,2 Mol) wasserfreiem Eisen-III-chlorid gelöst — gelöst in Nitromethan (70 ml Lösung) — zugetropft ; danach wurde noch 20 Stunden nachgerührt. Während der gesamten Zeitspanne wurde die Temperatur auf 20-25 °C (RT) gehalten.

Die dunkle Reaktionslösung wurde in Wasser gegossen, filtriert und nach dem Abtrennen die organische Phase neutral gewaschen. Dann wurde die Toluollösung mit festem $NaHCO_3$ und Bleicherde verrührt, filtriert und eingeengt. Die Rohausbeute betrug 320 g (= 94 % d. Th.).

Nach GC-Analyse bestand das Produkt zu über 97 % aus dem Isomerengemisch der Methylmononitrodiphenylmethane mit überwiegend in 4- oder 2-Stellung vorliegenden Methylgruppen.

b) 227 g (1,0 Mol) des Rohproduktes von a) wurden in 400 ml Methylenchlorid aufgenommen. Dazu wurden unter Rühren und Kühlen bei 20 °C langsam Nitriersäure zugegeben, bestehend aus einer Mischung von

142 g (2,2 Mol) 98 %iger $NHO_3$ und
220 g (2,2 Mol) 98 %iger $H_2SO_4$

Nach beendeter Zugabe wurde noch insgesamt 5,5 Stunden nachgerührt (1 Stunde bei 20 °C, 2 Stunden bei 30 °C und 2,5 Stunden bei 40 °C).

Danach wurde die Nitriersäure abgetrennt und die organische Phase analog Beispiel 1 b) aufgearbeitet. Der Rückstand betrug 287 g.

c) 250 g des Rückstandes von Stufe b) wurden in 600 ml Methanol aufgenommen und analog Beispiel 1 c) nach Zusatz von 20 g Raney-Nickel B reduziert. Nach dem Aufarbeiten verblieben 180 g Rückstand, der bei 0,133 3 mbar (0,1 Torr) destilliert wurde.

Nach einem Vorlauf von 8 g (110-140 °C) ging die Hauptfraktion (151 g) zwischen 140 und 280 °C über. Es verblieb ein Rückstand von 16 g.

Die gaschromatographische Analyse der Hauptfraktion ergab :

ca. 1,6 Gew.-% niedrigsiedende Amine (TDA)
ca. 31,0 Gew.-% Zweikerndiamine
ca. 60,0 Gew.-% Zweikerntriamine
ca. 8,0 Gew.-% höhersiedende Amine,

wobei die Di- und Triamine zu ca. 75 Gew.-% aus verbindungen bestanden, die an jedem aromatischem Ring mindestens eine Aminogruppe aufwiesen, und wobei die Aminogruppen in 2- und/oder 4-Stellung des keinen Methylsubstituenten aufweisenden Rings bzw. in 3- und/oder 5-Stellung des methylsubstituierten Ringes angeordnet waren, und wobei die Methylsubstituenten in 2-, 4- oder 6-Stellung angeordnet sind.

d) Die Haupfraktion (150 g) wurde in 1,5 l Monochlorbenzol gelöst und bei 0 °C unter Rühren und Kühlen zu einer Lösung von 500 g Phosgen in 1,5 l Monochlorbenzol langsam zugetropft. Anschließend wurde unter weiterem Durchleiten von Phosgen das Reaktionsgemisch langsam aufgeheizt und eine Stunde am Rückfluß gekocht.

Im Wasserstrahlvakuum wurde anschließend das Monochlorbenzol abgezogen und bei weiter reduziertem Druck (0,133 3 mbar (0,1 Torr)) das Isocyanat destilliert. Nach einem Vorlauf von ca. 10 ml gingen bei 145-180 °C 156 g nur noch aus Di- und Triisocyanaten bestehendes Isocyanatgemisch über. Als Destillationsrückstand verblieben 35 g.

Das Isocyanatgemisch wies einen NCO-Gehalt von 37,9 Gew.-% auf (Funktionalität 2,65).

## Beispiel 5 (4. Verfahren)

a) Unter Stickstoff wurden 18,4 kg (200 Mol) trockenes Toluol vorgelegt und mit 20 g wasserfreiem Eisen-III-chlorid versetzt. Unter Rühren wurden dazu bei 20-25 °C 2,53 kg Benzylchlorid zugetropft, wobei gasförmiger Chlorwasserstoff entwich.

Nach beendeter Zugabe wurde noch 30 Minuten nachgerührt, abgekühlt und dreimal mit je 5 l Wasser säurefrei gewaschen. Anschließend wurde aus der organischen Phase durch Destillation bei Normaldruck bis zu einer Sumpftemperatur von 150 °C das überschüssige Toluol abgetrennt.

Das verbleibende Kohlenwasserstoffgemisch (ca. 3,5 kg) bestand aus

ca. 90 Gew.-% 2-Kernisomeren
ca. 10 Gew.-% Mehrkernisomeren

Durch Destillation bei vermindertem Druck wurde zunächst die Zweikernfraktion (ca. 3,15 kg ; 78-80 °C/0,133 3 mbar (0,1 Torr)) abgetrennt und anschließend die Dreikernfraktion (280 g ; 150-175 °C/

0,133 3 mbar (0,1 Torr)) destilliert.

Die Zweikernfraktion bestand nach GC aus

40-42 Gew.-% 2-Methyldiphenylmethan
4- 5 Gew.-% 3-Methyldiphenylmethan
54-56 Gew.-% 4-Methyldiphenylmethan

b) 1 638 g (9 Mol) des nach a) erhaltenen Gemisches der Zweikernisomeren wurden in 3 600 ml Methylenchlorid bei Raumtemperatur vorgelegt.

Bei 25-30 °C wurden unter Rühren und Kühlen Nitriersäure zugegeben, bestehend aus

160 g (18 Mol) 98 %iger $HNO_3$ und
1 800 g (18 Mol) 98 %iger $H_2So_4$

Nach beendeter Zugabe wurde noch 15 Minuten nachgerührt und dann die Mischsäure abgetrennt. Anschließend wurde zu der organischen Phase bei 10-20 °C weitere Mischsäure bestehend aus

675 g (10,4 Mol) 98 % $HNO_3$ und
2 100 g (21 Mol) 98 %iger $H_2SO_4$

zugetropft und über Nacht bei Raumtemperatur gerührt.

Nach dem Abtrennen der organischen Phase wurde zweimal mit je 1,25 l Wasser, einmal mit 5 %iger Natriumcarbonatlösung und noch zweimal mit Wasser gewaschen. Anschließend wurde das Methylenchlorid bei Normaldruck bei 90 °C Sumpftemperatur abdestilliert und die Lösungsmittelreste mit Wasserdampf im Wasserstrahlvakuum bei 80 °C entwässert. Der Rückstand betrug 2 564 g Nitroprodukt.

c) 500 g des Rohproduktes der Nitrierstufe b) wurden in einem Gemisch aus 500 ml Methanol und 500 g Toluol aufgenommen, mit 50 g frischem Raney-Nickel (15 Gew.-% Eisengehalt) und 2 g festem Natriumcarbonat versetzt und bei 50 °C mit einem Wasserstoffdruck von 100 bar reduziert. Nach Beendigung der exothermen Reaktion wurde noch 1 Stunde bei 100 °C der Wasserstoffdruck auf 100 bar gehalten. Danach wurde entspannt, der Katalysator abfiltriert und Methanol und wasser abdestilliert.

Anschließend wurde der Rückstand (392 g) bei einem Druck von 0,133 3-0,266 6 mbar (0,1-0,2 Torr) bei einer Sumpftemperatur von 300 °C ausdestilliert, wobei 366 g Destillat und 22 g Rückstand erhalten wurden.

Nach GC-Analyse enthielt das Destillat (Mittelwert aus vier Ansätzen) :

8,6 Gew.-% niedrig siedende Amine
65,8 Gew.-% 2-Kerndiamine
25,6 Gew.-% 2-Kerntriamine

d) 500 g des Amingemisches (Destillat) von c) wurden bei 35-40 °C in 2,5 l Monochlorbenzol gelöst und bei 0 °C unter Rühren und Kühlen zu einer Lösung von 1,25 kg Phosgen in 2,5 l Monochlorbenzol langsam zugetropft. Anschließend wurde unter weiterem Durchleiten von Phosgen das Reaktionsgemisch langsam aufgeheizt bis das Monochlorbenzol am Rückfluß kochte und so eine Stunde lang gehalten.

Nach dem Abziehen des Monochlorbenzols im Wasserstrahlvakuum wurde der Rückstand zügig bei einem reduzierten Druck von 0,133 3 mbar (0,1 Torr) destilliert. Die Ausbeute betrug 559 g Destillat bei ca. 75 g Rückstand.

Das Rohdestillat wurde anschließend über eine 10 cm lange Kolonne langsam fraktioniert (70 g-Fraktionen) destilliert. Die einzelnen Fraktionen werden in nachstehender Tabelle beschrieben. Nach einem Vorlauf, der neben niedrigsiedenden Isocyanaten aus Nebenreaktionen ca. 25-30 Gew.-% Methyldiisocyanatodiphenylmethan enthielt, begann ab Fraktion 4 der NCO-Gehalt zu steigen und durch zunehmenden Gehalt an Triisocyanaten, die in der letzten Fraktion praktisch rein übergingen.

(Siehe Tabelle Seite 14 f.)

Tabelle

| Fraktion | Temp./Druck | NCO-Gehalt | Funktionalität |
|---|---|---|---|
| VL* (70 g) | 91-155 °C/0,133 3 mbar | | |
| 1 (70 g) | 155 | 31,8 Gew.-% | 2,0 |
| 2 (70 g) | — | 31,7 Gew.-% | 2,0 |
| 3 (70 g) | 170 °C/0,133 3 mbar | 31,7 Gew.-% | 2,0 |
| 4 (70 g) | 170-175 °C/0,133 3 mbar | 32,0 Gew.-% | 2,0 |
| 5 (70 g) | 176-180 °C/0,133 3 mbar | 36,1 Gew.-% | 2,5 |
| 6 (70 g) | 180-190 °C/0,133 3 mbar | 39,7 Gew.-% | 2,8 |
| 7 (58 g) | 190-205 °C/0,133 3 mbar | 41,2 Gew.-% | 3,0 |
| R (11 g) | | | |

* VL = Vorlauf

Beispiel 6 (4. Verfahren)

a) wie Beispiel 5a)

b) 910 g (5 Mol) des nach Beispiel 5a) erhaltenen Gemisches der Zweikernisomeren des Methyldiphenylmethans wurden in 2 000 ml Methylenchlorid bei 0-10 °C vorgelegt. Unter Rühren und Kühlen wurde bei dieser Temperatur Nitriersäure zugegeben, bestehend aus einer Mischung von

710 g (11,0 Mol) 98 %iger $HNO_3$ und
1 100 g (11,0 Mol) 98 %iger $H_2SO_4$

Bei 10 °C wurde nach beendeter Zugabe von 30 Minuten nachgerührt und dann die Mischsäure abgetrennt. Die organische Phase wurde zweimal mit je 1 000 ml Wasser, einmal mit 5 %iger Natriumcarbonatlösung und noch zweimal mit Wasser gewaschen. Anschließend wurde das Methylenchlorid bei Normaldruck bis 90 °C Sumpftemperatur abdestilliert und die Lösungsmittelreste mit Wasserdampf im Wasserstrahlvakuum bei 80 °C entwässert. Der Rückstand (1 290 g) enthielt :

7-8 Gew.-% Nitrotoluol
< 1 Gew.-% Mononitro }
92,6 Gew.-% Dinitro } -methyldiphenylmethan
< 1 Gew.-% Tri- und Tetranitro }

Die Dinitrofraktion bestand aus ca. 18 Gew.-% dinitriertem 2-, 4- oder 6-Methyldiphenylmethanen mit in 3,2'-Stellung angeordneten Nitrogruppen, ca. 55 Gew.-% entsprechender 3,4'-Dinitroverbindungen und ca. 27 Gew.-% an anderen Isomeren.

1 000 g des gemäß b) erhaltenen Rohgemisches der Nitroverbindungen wurden bei Raumtemperatur mit 1 l Ethanol zu einem Kristallbrei verrührt und auf einer Nutsche abgesaugt. Der Rückstand wurde anschließend einmal aus Isopropanol umkristallisiert. Der Rückstand des Kristallisationsschrittes (250 g = 0,92 Mol) bestand aus 3,4'-Dinitro-4-methyldiphenylmethan in ca. 98 %iger Reinheit (Fp. 146-147 °C).

b) 136 g (0,5 Mol) 4-Methyl-3,4'-dinitrodiphenylmethan wurden analog Beispiel 1 b) erneut mit Nitriersäure, bestehend aus

37 g (0,57 Mol) 98 %iger $HNO_3$ und
57 g (0,57 Mol) 98 %iger $H_2SO_4$

in 300 ml Methylenchlorid bei 20 °C und nach beendeter Zugabe bei 25-30 °C umgesetzt und analog Beispiel 1 b) aufgearbeitet und ergaben einen Rückstand von 154 g.

c) 150 g des Rückstandes von b) wurden analog Beispiel 1 c) zu den Aminoverbindungen reduziert und aufgearbeitet. Die Rohausbeute betrug 101 g und wurde anschließend bei 0,133 3 mbar (0,1 Torr) Ölpumpenvakuum destilliert.

Nach einem Vorlauf von ca. 5 g, der aus niedrigsiedenden Verbindungen bestand, wurde zwischen 140 °C und 280 °C eine Hauptfraktion von 86 g aufgefangen, während im Destillationskolben ein Rückstand von 10 g verblieb.

Nach GC bestand die Hauptfraktion aus :

3,6 Gew.-% 2,4-Diaminotoluol
34,5 Gew.-% 4-Methyl-3,4'-diaminodiphenylmethan

3,1 Gew.-% 4-Methyl-3,2,4'-triaminodiphenylmethan
57,1 Gew.-% 4-Methyl-3,5,4'-triaminodiphenylmethan
< 2 Gew.-% höhersiedende Aminverbindungen

d) Das Destillat von c) (85 g) wurde analog Beispiel 1 d) mit Phosgen umgesetzt und aufgearbeitet. Das Rohisocyanat (103 g) wurde bei 0,533 2 mbar (0,4 Torr) destillativ aufgetrennt in

| | |
|---|---|
| Vorlauf (70-155 °C) : | 10,3 g |
| Hauptfraktion (155-180 °C) : | 83,6 g |
| Rückstand : | 7 g |

Die Hauptfraktion bestand nach GC-Analyse aus :

40,0 Gew.-% 2-Kern-diisocyanat
57,5 Gew.-% 2-Kern-triisocyanat

und wurde fraktioniert destilliert in

Fraktion 1 (155-175 °C/0,266 6 mbar) : 51,3 g
Fraktion 2 (175-176 °C/0,266 6 mbar) : 26,4 g

Fraktion 1 bestand nach GC-Analyse aus :

50 Gew.-% 4-Methyl-3,4'-diisocyanatodiphenylmethan
50 Gew.-% 4-Methyl-3,5,4'-triisocyanatodiphenylmethan.

Fraktion 2 bestand nach GC-Analyse aus :

> 99 Gew.-% 4-Methyl-3,5,4'-triisocyanatodiphenylmethan

(blaßgelbe Nadeln, Fp. 70 °C, NCO-Gehalt von 41,2 Gew.-%).

Beispiel 7 (4. Verfahren)

a) wie Beispiel 5a)

b) 1 400 ml 83 %iger Schwefelsäure wurden bei 28-30 °C vorgelegt. Dazu wurden unter Rühren und Kühlen gleichzeitig und in konstantem stöchiometrischem Verhältnis von 2,5 : 1 zudosiert :

3 490 g (21,25 Mol) einer Mischsäure, bestehend aus
2 125 g 98 %iger Schwefelsäure und
1 365 g 98 %iger Salpetersäure, und
1 547 g (8,5 Mol) des Isomerengemisches der Methyldiphenylmethane von a).

Nach beendeter Zugabe wurde 0,5 Stunden ohne Kühlung gerührt, dann langsam auf 100 °C aufgeheizt, 2 Stunden bei 100 °C gehalten und dann bei dieser Temperatur in eine Schwefelsäurephase und eine produktphase getrennt.

Di Produktphase wurde bei 80 °C unter Rühren erst mit 1 000 ml Wasser und danach mit 5 000 ml Toluol versetzt. Die wäßrige Phase wurde abgetrennt und die organische Phase erst zweimal mit je 1 500 ml 3 %iger wäßriger Sodalösung und anschließend zweimal mit je 1 000 ml Wasser gewaschen.

Aus der abgetrennten organischen Phase wurde erst bei Normaldruck die Hauptmenge und dann im Wasserstrahlvakuum der Rest des Toluols abgetrennt, es verblieb ein Rückstand von 2 200 g Nitroprodukt.

c) 200 g des Nitroprodukts von b) wurden in 800 ml Methanol in Gegenwart von 20 g Raney-Nickel bei 50 °C und 50 Bar Wasserstoffdruck bis zur Druckkonstanz hydriert. Nach dem Absaugen des Katalysators wurde das Lösungsmittel abdestilliert. Der Rückstand (147 g) hatte nach GC die folgende Zusammensetzung :

1,1 Gew.-% Aminotoluole
4,4 Gew.-% Diaminotoluole
74,8 Gew.-% Diamino-methyldiphenylmethane
19,7 Gew.-% Triamino-methyldiphenylmethane.

d) 145 g des Rohamins von c) wurden in 1,4 l Monochlorbenzol gelöst und bei 0 °C unter rühren und Kühlen zu einer Lösung von 400 g Phosgen in 1,4 l tr. Monochlorbenzol langsam zugetropft, unter weiterem Durchleiten von phsogen aufgeheizt und drei Stunden unter Rücktluß nachphosgeniert.

Anschließend wurde das Phosgen ausgeblasen und das Lösungsmittel eingeengt.

Der Rückstand (182 g) wurde an der Ölpumpe destilliert. Dabei gingen nach einem Verlauf von 13 g (0,133 3 mbar/90-150 °C) eine Hauptfraktion von 161 g mit einem Siedepunkt von 150-205 °C/ 0,133 3 mbar (0,1 Torr) über (NCO-Gehalt 33,9 Gew.-%, bezogen auf MG 42). Nach GC hatte das Destillat die Zusammensetzung :

78 Gew.-% Diisocyanatomethyldiphenylmethane
22 Gew.-% Triisocyanatomethyldiphenylmethane.

**Ansprüche**

1. Gegebenenfalls im Gemisch mit bis zu 40 Gew.-%, bezogen auf Gesamtgemisch, an anderen alkylsubstituierten Polyisocyanatodiphenylmethan-Isomeren vorliegende Gemische von Polyisocyanaten der Formel

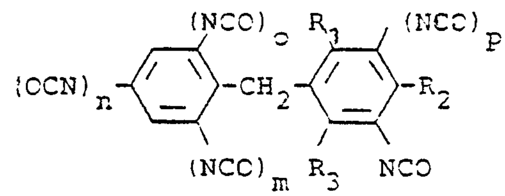

in welcher jeder der beiden aromatischen Ringe mindestens eine Isocyanatgruppe aufweist und wobei

$R_1$, $R_2$ und $R_3$ jeweils gleich oder verschieden sind und Wasserstoff oder eine Methyl- oder Ethylgruppe bedeuten, mit der Einschränkung, daß jeweils zwei der Reste $R_1$, $R_2$ und $R_3$ für Wasserstoff stehen und

m, n, o und p jeweils für 0 oder 1 stehen, wobei im Falle von m, n, o und/oder p = 0 die freie Valenz durch ein Wasserstoffatom abgesättigt ist, und wobei die Summe m + n + o + p im statistischen Mittel 1,2 bis 1,7 beträgt.

2. Verwendung der Polyisocyanatgemische gemäß Anspruch 1 als Isocyanatkomponente bei der Herstellung von Polyurethankunststoffen nach dem Isocyanat-Polyadditions-verfahren.

**Claims**

1. Mixtures of polyisocyanates of the formula

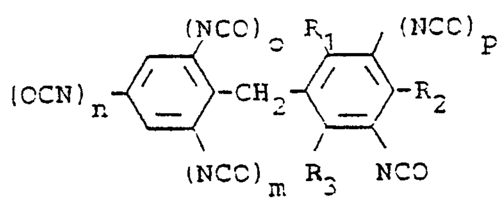

in which each of the two aromatic rings contains at least one isocyanate group and wherein

$R_1$, $R_2$ and $R_3$ are in each case identical or different and denote hydrogen or a methyl or ethyl group, with the proviso that in each case two of the radicals $R_1$, $R_2$ and $R_3$ represent hydrogen and

m, n, o and p each represent 0 or 1 ; when m, n, o and/or p = 0 the free valency is saturated by a hydrogen atom, the sum of m + n + o + p on a statistical average being 1.2 to 1.7, which are optionally mixed with up to 40 % by weight, based on the total mixture, of other alkyl-substituted polyisocyanatodiphenylmethane isomers.

2. Use of the polyisocyanate mixtures according to Claim 1 as an isocyanate component in the production of polyurethane plastics by the isocyanate polyaddition process.

**Revendications**

1. Mélanges de polyisocyanates de formule

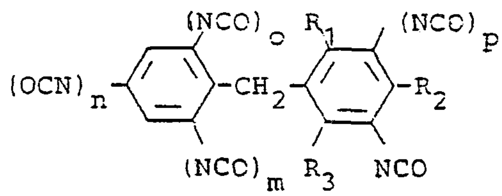

dans laquelle chacun des deux noyaux aromatiques présente au moins un groupe isocyanate et

$R_1$, $R_2$ et $R_3$ sont tous égaux ou différents et représentent de l'hydrogène ou un groupe méthyle ou éthyle, sous réserve que dans chaque cas deux des restes $R_1$, $R_2$ et $R_3$ représentent de l'hydrogène et

m, n, o et p sont chacun égaux à 0 ou à 1 et, dans le cas où m, n, o et/ou p sont égaux à 0, la valence libre est saturée par un atome d'hydrogène, et la somme m + n + o + p s'élève en moyenne statistique à une valeur de 1,2 à 1,7,

éventuellement en mélange avec jusqu'à 40 % en poids, par rapport au mélange total, d'autres isomères de polyisocyanatodiphénylméthane à substituants alkyle.

2. Utilisation de mélanges de polyisocyanates suivant la revendication 1 comme composant isocyanate dans la production de matières plastiques du type polyuréthanne par le procédé de polyaddition d'isocyanate.